Europäisches Patentamt

European Patent Office

Office européen des brevets

(19)

(11) **EP 0 505 442 B1**

(12) **EUROPEAN PATENT SPECIFICATION**

(45) Date of publication and mention
of the grant of the patent:
**05.02.1997 Bulletin 1997/06**

(21) Application number: **91901246.8**

(22) Date of filing: **13.12.1990**

(51) Int Cl.[6]: **G01N 27/26**, G01N 27/49

(86) International application number:
**PCT/US90/07345**

(87) International publication number:
**WO 91/09302 (27.06.1991 Gazette 1991/14)**

(54) **METHOD FOR INCREASING THE SERVICE LIFE OF AN IMPLANTABLE SENSOR**

VERFAHREN ZUR ERHÖHUNG DER FUNKTIONSLEBENSDAUER EINES IMPLANTIERBAREN FÜHLERS

PROCEDE PERMETTANT D'ALLONGER LA VIE UTILE D'UN CAPTEUR IMPLANTABLE

(84) Designated Contracting States:
**AT BE CH DE DK ES FR GB GR IT LI LU NL SE**

(30) Priority: **14.12.1989 US 450852**

(43) Date of publication of application:
**30.09.1992 Bulletin 1992/40**

(73) Proprietor: **THE REGENTS OF THE UNIVERSITY OF CALIFORNIA**
**Berkeley, California 94720 (US)**

(72) Inventors:
• **GOUGH, David, A.**
  **Cardiff-By-The-Sea, CA 92007 (US)**
• **LUCISANO, Joseph, Y.**
  **Saugus, CA 91350 (US)**

(74) Representative: **Wilson, Nicholas Martin et al**
**WITHERS & ROGERS**
**4 Dyer's Buildings**
**Holborn**
**London EC1N 2JT (GB)**

(56) References cited:
US-A- 2 508 523     US-A- 2 563 062
US-A- 2 805 191     US-A- 2 864 750
US-A- 2 998 371     US-A- 3 246 235
US-A- 3 249 520     US-A- 3 300 345
US-A- 3 308 046     US-A- 3 458 421
US-A- 3 616 412     US-A- 4 036 716
US-A- 4 088 550     US-A- 4 306 952
US-A- 4 650 547     US-A- 4 671 288
US-A- 4 703 756     US-A- 4 781 798
US-A- 4 830 713

• JOSEPH Y. LUCISANO et al., "In Vitro Stability of an Oxygen Sensor", Reprinted from Analytical Chemistry, Volume 59, (1987), pp. 736-739.
• JOSEPH Y. LUCISANO, "Development of an Implantable Glucose Sensor: Stability of the Oxygen Electrode and Analysis of the Transient Response", dissertation, Univ. of Calif. (San Diego), pp. 8-10, 26-30, 34-36, 96 and 97. (Dec. 15, 1988). See pages 34-36 and 43.

**EP 0 505 442 B1**

## Description

### BACKGROUND OF THE INVENTION

### FIELD OF THE INVENTION

The invention herein relates to implantable sensors for measuring the concentrations of chemical species in bodily fluids. More particularly it relates to methods of extending the service life of such sensors.

### DESCRIPTION OF THE PRIOR ART

The electrochemical oxygen sensor has been a powerful tool for revealing the role of oxygen in biological systems. Application of this sensor has been the main experimental methodology in many thousands of studies over more than 40 years. Virtually all, however, have been studies in which the sensor is used for a period of only a few days at most before recalibration is necessary. More recently there has been developed a stable oxygen sensor that is suitable for continuous application in long-term monitoring situations without the need of frequent recalibration. Such a sensor makes possible certain important oxygen monitoring applications that were not previously feasible.

This type of sensor, and its application as a component of an enzyme electrode-based system for continuously monitoring glucose, have been described in several prior patents: U.S. Patents Nos. 4,650,547; 4,671,288; 4,703,756 and 4,781,798. The glucose monitoring system requires two oxygen such sensors, one coupled to immobilized enzymes to detect oxygen modulated by the enzyme reaction, and the other to monitor the background oxygen concentration.

When a noble metal working electrode (usually platinum or gold) is immersed in an electrically conductive medium and held at a potential sufficiently cathodic with respect to an appropriate reference electrode, oxygen molecules in contact with the surface are reduced and an oxygen diffusion gradient is established, resulting in an electrical current. The current passes between the working and reference electrodes if a two-electrode system is used, or mainly between the working electrode and an indifferent counter electrode separate from the reference electrode when a three-electrode system is used. This phenomenon was observed and reported in the 19th century. Under certain conditions, the reduction current may be related to oxygen concentration in the medium. A membrane permeable to oxygen can be placed over the electrodes to separate them from the analyte medium and provide the appropriate conditions for oxygen mass transfer and electrochemical oxygen reduction. This principle forms the basis of amperometric (current-measuring) electrochemical oxygen sensors. Three electrode amperometric sensors are often referred to as potentiometric sensors, since they are operated by potentiostatic instrumentation. It is recognized that the application of this principle will be influenced by factors such as: impurities in the media; Ph and reaction intermediates; oxygen dissolved interstitially in the metallic electrode; the nature of the background electrolyte; and the degree of electrode surface oxide coverage.

The reaction pathways are complex. They include the kinetics of oxygen adsorption on and into the electrode and the formation of multiple metal-oxygen complexes involving short-lived intermediates. A detailed consideration of these pathways is not required here, but simplified models of the electrode reactions that are believed to explain many aspects are helpful in understanding the invention. One widely accepted mechanism in acidic media is the following two-step process:

$$O_2 + 2H^+ + 2e^- \rightarrow H_2O_2 \qquad (1.1)$$

$$H_2O_2 + 2H^+ + 2e^- \rightarrow 2H_2O \qquad (1.2)$$

In alkaline media, a similar process has been proposed:

$$O_2 + H_2O + 2e^- \rightarrow HO_2^- + OH^- \qquad (2.1)$$

$$HO_2^- + H_2O + 2e^- \rightarrow 3OH^- \qquad (2.2)$$

$HO_2$ is the ionized form of $H_2O_2$ that is present in alkaline media. These two sets of equations indicate that oxygen reduction can proceed by either a 2 or 4 electron process on platinum in aqueous solutions.

When the electrode is polarized within a certain cathodic range, the rate of electrochemical reaction is sufficiently rapid that the process becomes mass transfer limited and determined by the mass transfer limitation of the membrane and analyte medium. This results in a current "plateau," in which there is relatively little variation in current with applied potential. The electrode can be most easily operated as part of a sensor in this potential range.

Certain three-electrode sensors of this type, especially those described in the aforesaid U.S. Patents, have been shown to have long-term stability under well-defined in vitro and in vivo conditions. It has been found, however, that after a period of operation, such sensors tend to fall in one or the other of two characteristic modes. In most cases, the current rose abruptly and returned to the original value several times over the period of a few hours before finally remaining at a high, off-scale value. In other sensors, the current suddenly began to drift downward and fell over a period of several weeks.

An article in Analytical Chemistry, 1987, 59, 736 entitled "In Vitro Stability of an Oxygen Sensor" discussed the problem of extending the life of implantable three electrode sensors. No solutions were suggested in that article.

It would be of significant value to have a method for preventing or deferring such failures, since such would be expected to substantially increase the operating service life of the sensors. It is therefore an object of this invention to provide such a method.

## SUMMARY OF THE INVENTION

The invention herein is a method of extending the service life of implantable sensors containing corrodible electrodes, which comprises, in a sensor circuit including a sensor having a corrodible reference electrode, at least one noble metal cathodic working electrode, and at least one noble metal anodic counter electrode maintained at a low impedance, operating the sensor circuit in a first electrical configuration in which both said reference electrode and said working electrode are in a first electrical state, and prior to occurrence of sensor failure, switching the said sensor circuit to a second electrical configuration in which the electrical state of at least one of said reference electrode and said working electrode is changed to a second electrical state and continuing operation of said sensor in said second electrical configuration for a period of time extending beyond the time at which said sensor would have failed had said first electrical configuration of the sensor circuit been maintained.

In specific embodiments, the change in the electrical circuit configuration may comprise: increasing the impedance at the reference electrode, preferably while shielding the electrode against effects of stray capacitance; electrically reversing the two electrodes; having a plurality of working and/or reference electrodes present, with only one of each active at any time, and sequentially replacing the operating electrode in the sensor circuitry with a second like electrode which was previously inactive; periodically reversing the electrical potential to the working and the reference electrodes and maintaining that reversed state for an extended time; and/or causing a small, continuous cathodic current to pass through the reference electrode.

## BRIEF DESCRIPTION OF THE DRAWINGS

FIGURE 1 is a perspective view, partially cut away, illustrating the basic sensor design applicable to this invention.

FIGURE 2 is an schematic electrical diagram showing the basic circuitry of the sensors applicable to this invention.

FIGURE 3 is an schematic electrical diagram showing the circuitry of an embodiment of this invention in which a plurality of working and/or reference electrodes is used.

FIGURE 4 is an schematic electrical diagram showing the circuitry of an embodiment of this invention in which the polarities of the working and reference electrodes are reversible.

FIGURE 5 is an schematic electrical diagram showing the circuitry of an embodiment of this invention in which the functions of the working and counter electrodes are reversible.

FIGURE 6 is a schematic electrical diagram showing the circuitry of an embodiment of this invention in which a small cathodic current is caused to pass through the reference electrode.

## DETAILED DESCRIPTION OF THE INVENTION

This invention is the result of discoveries made during the study of the failure mechanisms of the sensors described in the above-cited patents. It is therefore important to describe the normal operation of such sensors and the discoveries made about the failure modes, so that the method of this invention and its specific embodiments for extending the service life of such sensors will be fully understood.

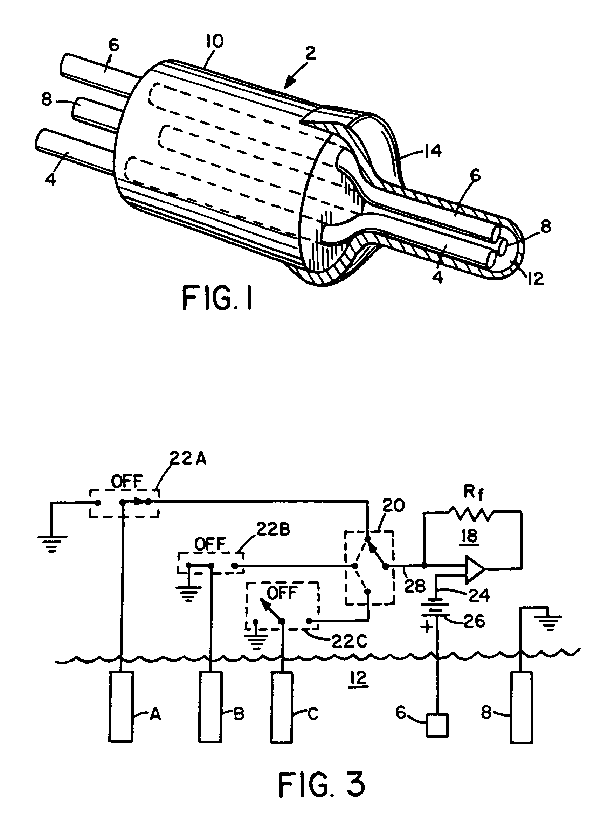

The basic sensor is illustrated in Figure 1. The platinum working electrode 6, the silver/silver chloride reference electrode 4 and the platinum counter electrode 8 are fine wires embedded in an epoxy resin or glass cylinder 10 and connected to more substantial lead wires not shown. The active electrodes extend from one end of the insulating cylinder and the lead wires extend from the opposite end. The electrodes are in electrolytic contact through an aqueous electrolyte gel 12 and encased by an outer hydrophobic layer 16. The thin, cylindrical shape of the active region of this oxygen sensor readily allows for its inclusion in the two-dimensional glucose sensor described in the above-cited patents. Alternatively, one or more of the electrodes may take the form of disks instead of cylinders, with the exposed surface flush with the surface of the insulating cylinder.

Test sensors were disassembled after a period of operation (commonly 120 days) for detailed microscopic examination. In a typical analysis, it was found that the reference electrode (originally formed from a solid silver wire, of constant 0.075 mm diameter) had partially dissolved, had become porous and had significantly corroded, with only approximately one half of its original material remaining at the time of examination. The degree of corrosion observed was approximately proportional to the time of operation of the sensor. Similar examinations of the working electrode showed that it had acquired a granular surface structure with use, which, according to X-ray elemental analysis, was a deposition of a thin layer of silver.

In the cases of gradual sensor failure, the original signal could be restored by appropriate polarization treatment of the working electrode. In cases of abrupt sensor failure, a dendritic silver structure had formed a contact between the working and reference electrodes, apparently growing from the working electrode. In all cases, the counter electrode retained its original surface composition and microstructure and otherwise showed no change.

The microscopic examination indicates that various processes are believed to occur during operation. The working electrode gradually becomes coated with silver as a result of being cathodically polarized with respect to the silver source, the reference electrode. At that point, the oxygen-reduction process no longer occurs on the underlying platinum surfaces but continues unaffected on the deposited outer silver coating. The deposition of silver apparently does not affect the signal as long as no dendritic contact with the other electrodes is made. The transfer of silver from the reference electrode was, however, somewhat surprising because the reference electrode is maintained at very high impedance by the potentiostat circuit (>$10^{12}$ $\Omega$ based on the input impedance of the reference operational amplifier). Calculations show that a leakage current of $10^{-12}$ A between the two electrodes is sufficient in some cases to account for the small amount of material transferred. Transient local capacitive currents as a result of inadequate shielding of the lead wires may also have played a role. These currents, induced by external electromagnetic fields, were observed to reach peak values as high as 5 x $10^{-9}$ A in other experiments involving electrodes placed in electrically "noisy" environments.

In most cases, sensor lifetime was limited by corrosion of the reference electrode, with failure occurring when a dendritic contact (a deposit formed from dissolved silver) ultimately connected the working and reference electrodes.

Based on these observations, the various embodiments of the method of this invention were developed. All rely on the basic principle of changing at least one of the working electrode and the reference electrode from the electrical state in which it is originally operated to a second electrical state, and then continuing the operation of the sensor with the electrode or electrodes operating in that second electrical state. It is believed that the prolongation of the service life of the sensors operated according to the method of this invention is due to the change in electrical state reversing or slowing the transfer of metal from one electrode to the other and/or the formation of dendritic structures of metal between the electrodes.

One embodiment of the present method comprises increasing the input impedance at the reference electrode, preferably while shielding the electrode to counteract the effects of stray capacitance which would cause local currents. It is recognized, however, that since the initial impedance is already high (>$10^{12}$ $\Omega$) this embodiment, while showing a definite effect, is somewhat limited in its long-term potential and is therefore less preferred than other embodiments described.

A second embodiment comprises, prior to formation of any dendritic structure bridging the two electrodes, reversing the electrical connections to the working and counter electrodes, as shown in Figure 3, so that each assumes the prior operating role of the other. This will cause the new counter electrode to become cleaned of its accumulated layer of deposited silver and silver to become deposited on the new working electrode. This reversal can be repeated as long as the reference electrode remains functional and contains a sufficient amount of silver. This would require a relatively large reference electrode.

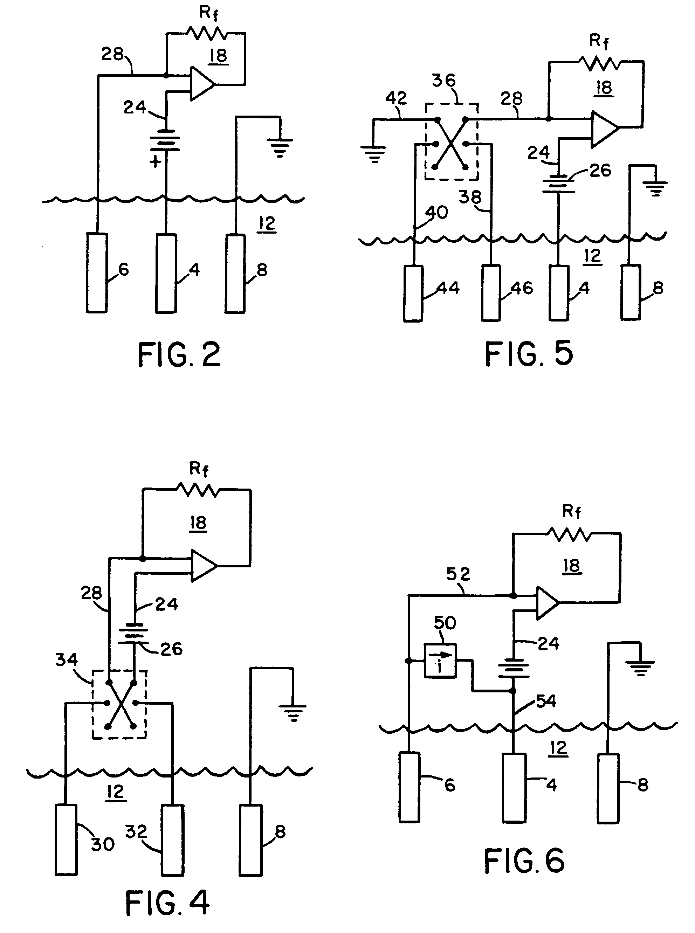

A third embodiment comprises including in the sensor structure a plurality of working and/or reference electrodes, as shown in Figure 4. Only one of each type of electrode would be operative as a working or reference electrode at any one time, and all electrodes of each type would be designed so that they could be connected sequentially into the sensor circuit. Thus as each working and/or reference electrode approached the limit of its service life because of metal deposition or removal, a second previously unused electrode could be switched into its place. In a preferred version of this embodiment, the extra working electrodes are initially incorporated into the sensor circuitry as counter electrodes and each operates as such until such time as it is needed as a replacement working electrode. This will enable the reserve working electrodes to maintain a deposit-free surface until each is used as a replacement for a previous working electrode.

With respect to the reserve reference electrodes, these would be most effective if they were relatively large.

The fourth embodiment of this present method comprises periodically reversing the electrodes' polarization so as to drive electrodeposited metal (usually silver) from working electrode back to the reference electrode. This can be accomplished by reversing the polarity of the circuit such that the silver reference electrode becomes cathodic and the working electrode becomes anodic for a period of time sufficient to pass the required number of coulombs of silver from the working electrode to the reference electrode.

The fifth embodiment of this present method comprises employing means to pass a small, continuous cathodic current through the reference electrode. This current, while not large enough to cause significant polarization of the electrode, functions to prevent corrosion of the electrode material.

It will be recognized that these embodiments may be used in various combinations with each other, and that such combinations may produce further extended service lifetimes. For example, a plurality of working and/or reference electrodes may be present and may be switched into the system, while at the same time the service life of each individual electrode may be extended by reversing the electrode's polarization periodically. This should result in a extension of the service life of each individual electrode and thus extend the time between the necessary replacements of electrodes from the plural supply of electrodes. Also, any reference electrode currently in use may be impressed with a small cathodic current to reduce its rate of corrosion. Thus the cumulative effect of the combination of embodiments is a greatly extended service life for the sensor itself, and much longer intervals between implantations of fresh sensors.

Examination of the Figures of the drawings will further explain the embodiments of this invention. Referring first to Figure 1, the basic sensor 2 used in the present method is illustrated. There are three electrodes, the reference electrode 4, the working electrode 6 and the counter electrode 8. The reference electrode 4 is a silver/silver chloride electrode while the working electrode 6 and counter electrode 8 are noble metal (preferably platinum) electrodes. The three electrodes are formed of fine wire embedded in a glass or epoxy cylindrical housing 10 and are connected to more substantial lead wires (not shown). The electrodes are in electrical contact through an aqueous electrolyte gel 12. The electrodes 4, 6 and 8 are clustered outside of the housing 10 in a narrowed or necked region 14 which is covered by a hydrophobic oxygen permeable layer 16 of a material such as silicone rubber.

The sensor 2 is operated on the classical potentiostatic principle which is illustrated schematically in Figure 2. As with other types of sensors, oxygen is electrochemically reduced at the surface of the platinum working electrode 6, generating an electrode current that is proportional to the oxygen flux. The potential of the platinum working electrode is specified with respect to the silver/silver chloride reference electrode 4. In this mode of operation however, the reference electrode 4 is maintained electronically at a very high impedance to avoid significant current uptake. The main current passes to the inert counter electrode 8, which is maintained at low impedance. The operational amplifier circuitry 18 maintains the desired potential between the working and reference electrodes 6 and 4 by applying the appropriate potential between the working and counter electrodes 6 and 8. The voltage developed across the feedback resistor $R_f$ is proportional to the electrode current. This voltage is measured and processed by other circuitry not shown.

This three-electrode system provides separate electrodes for the two functions carried out by the anode in previous designs and has the advantage of directing very little current to the reference electrode 4. It makes possible the use of much larger ratios of the area of working electrode 6 to that of counter electrode 8, thereby producing larger currents. This makes signal amplification and noise reduction less critical in sensors of small overall size.

Embodiments of the method are illustrated graphically in Figures 3-6. Figure 3 shows an embodiment in which there are a plurality of working electrodes which can be switched sequentially into the system. Three electrodes (labeled A, B and C) comprise the working and reference electrodes. In the version shown in Figure 3, electrode A is wired as the working electrode, electrode B is wired as an extra counter electrode (along with regular counter electrode 8) and electrode C is out of the circuitry. The selection of which electrode is the working electrode at any given time is by means of the combination of 3-pole, sequential throw switch 20 and the individual SPDT-center off switches 22 (respectively 22A, 22B and 22C in each path). As each working electrode becomes deteriorated or subject to coating or bridging, it is switched out of the circuit by opening its switch 22 (i.e., moving the switch to the center-off position as at 22C), moving the switch 20 to the next sequential position and closing that next electrode's switch 22 as shown at 22A. While the remaining fresh electrodes can be left out of the system (by having their switches 22 left open) if desired, it is preferred to have them temporarily wired into the circuit as additional counter electrodes as shown with electrode B and switch 22B to prevent them from becoming coated with metal deposits.

It will be apparent that Figure 3 also illustrates the type of circuitry necessary for having a plurality of switchable reference electrodes. In this case the switch 20 would be placed in line 24 between the battery 26 and the plurality of electrodes. Each of the switches 22 could be SPST switches which would all be open except for the particular electrode which was then in use, since there is no purpose in having the extra electrodes alternatively serve as counter electrodes. One can also combine the presence of bcth extra working and reference electrodes by having separate switches 20 in each line 24 and 28 and sequencing the two parts of the circuit independently as needed.

While only three electrodes are shown in Figure 3, it will be evident that there can be any number, limited only by the number of electrode wires which can conveniently be clustered in the sensor 2 in combination with the electrodes 4, 6 and 8.

Figure 4 illustrates the embodiment where the polarity of the working and reference electrodes are reversible. In this case lines 24 and 28 lead into DPDT switch 34 which is wired such that throwing the switch reverses the leads to electrodes 30 and 32. Thus the electrodes 30 and 32 are connected alternatively as the working electrode in line 28 or the reference electrode in line 24.

Figure 5 illustrates the embodiment where the functions of the working and counter electrodes are reversible. In this case lines 28 and 42 (the latter from ground 48) lead into DPDT switch 36 which is wired such that throwing the switch reverses the connections between lines 28 and 42 and the leads 38 and 40 from electrodes 44 and 46. Thus the electrodes 44 and 46 are connected alternatively as the working electrode in line 28 or the counter electrode grounded through line 42.

Figure 6 illustrates the embodiment where a controlled current source 50 is placed between the working electrode lead 52 and the reference electrode lead 54. This current source functions to pass a small, continuous cathodic current through the reference electrode 6.

It will be recognized that the current source could be also placed in other areas of the circuit and achieve the same result.

The following examples will illustrate the sensors to which the service life extension method and embodiments of this invention can be applied. Electrodes were made by welding a small segment of 0.003 or 0.005-in.-diameter platinum or silver wire to one end of a long, PTFE-insulated stainless steel lead wire. The welded regions of two such platinum electrodes and one silver electrode were then encapsulated individually in the lumens of a short segment of multibore borosilicate glass tubing (0.010-in.-i.d., 0.062-in.-o.d.; Friedrich and Dimmock, Inc.) so that the electrodes and lead wires extended from opposite ends of the glass housing. A bisphenol A/epichlorohydrin-based epoxy resin (Stycast 1266, Emerson and Cuming, Inc.) was used for the encapsulation. Electrodes were carefully bent to the parallel arrangement shown in Figure 1 and trimmed to a length of 0.02-0.10 in. The working and counter electrodes were platinized to a roughness factor of approximately 800, as estimated by anodic hydrogen stripping. The electrolyte gel was formed around the electrodes by dipping the end of the assembly in a 10-20% solution of poly(hydroxyethyl methacrylate) (Polysciences, Inc.) in methanol, allowing the solvent to evaporate, and hydrating with electrolyte. The electrolyte was 0.01 M phosphate buffer, pH 7.3, containing 0.01 M KCl. The gel filled in the spaces between the electrodes and provided a thin coating on the outer aspects. After drying, the outer hydrophobic layer was formed by dipping the end of the assembly in a solution of 25% silicone rubber (RTV 3140, Dow Corning Corp.) in toluene. The solvent was evaporated and the silicone rubber allowed to cure. This produced a layer approximately 10-25 µm thick. The gel could be dehydrated and rehydrated by exposure to an aqueous sample without loss of activity. The assembly was then fixed in a silicone rubber tube (0.040-in.-i.d., 0.085-in.-o.d., Dow Corning Corp.) in such a way that the lead wires were extended inside the tube and the active electrode region occupied one end. This recessed design presents an annular space around the electrodes which may be filled with an enzyme gel for enzyme electrode applications. The annular cavity was filled with silicone rubber (RTV 3140) or the tube was trimmed to expose the hydrophobic membrane-covered electrode assembly. The space surrounding the lead wires was filled with silicone rubber (RTV 615, General Electric Co.) to provide mechanical strength. A miniature electrical connector (Microtech, Inc.) was attached to the lead wires at their exit from the tube. This simple fabrication approach typically gave a high yield of sturdy, functional sensors.

Sensors were checked for uniformity prior to long-term testing. The integrity of the silicone rubber coating was determined by measuring the resistance with respect to an external electrode using a high impedance electrometer (Keithley Instruments Co., Model 616).

Sensors with an apparent resistance of 10 x $10^9$ $\Omega$ or greater could be used in complex media without interference from diffusible polar solutes and were considered to have an effective barrier. Sensors with significantly lower apparent resistance were recoated. The background current in the absence of oxygen was measured and determined to be insignificant. Linearity of the response over a physiologic oxygen concentration range was verified by exposing sensors to oxygen concentrations ranging from 0.02 to 0.24 mM, made by equilibrating buffer solutions with analyzed gas mixtures of 2, 5, 10 and 21% oxygen. Sensors typically required approximately one minute to return to steady-state after a step change in oxygen concentration.

Sensors were evaluated for stability in sealed, thermostated vessels at 37°C, containing 0.01 M phosphate buffer, pH 7.3. Solutions were maintained at desired oxygen concentrations by equilibration with analyzed gas mixtures. The concentration was changed at intervals of several days to demonstrate sensitivity. Electrode current was recorded continuously.

Six sensors were operated continuously in a sealed, thermostated vessel at 37°C, containing 0.01 M phosphate buffer, pH 7.3. The oxygen concentration was maintained at atmospheric levels by equilibration with filtered room air. Some sensors incorporated specific design modifications described below to determine their influence on reference electrode degradation. Sensors were otherwise identical to that shown in Figure 1. Five sensors were disassembled after 70 days of continuous operation. One sensor was disassembled after 10 days of continuous operation. Reference electrodes were removed from the sensors and scanning electron microscopy was used to assess the extent of reference electrode degradation in each case. All reference electrodes were originally formed from solid, 0.075-mm-diameter silver wire.

A reference electrode was taken from a sensor that was subjected to the test conditions for 70 days but was not connected to any external circuitry to provide polarization of the working electrode. In this case, all electrodes were maintained at "open circuit" and were therefore not capable of sustaining DC current. This electrode served as a control in the experiment, since any corrosion in this case would be the result of simple electrolyte-silver interaction or passive noise pickup in the lead wires. Inspection by micrograph revealed little or no corrosion of this electrode.

Analysis by micrographs was made of reference electrodes taken from sensors that employed unshielded and shielded reference electrode lead wires, respectively. Both of these sensors were operated continuously for 70 days prior to disassembly. The reference electrode from the unshielded sensor exhibited substantially more corrosion than that from the shielded sensor. However, even the electrode in the shielded case showed significant corrosion over 20-30% of its surface. This indicates that lead wire shielding, although helpful in reducing corrosion, does not eliminate it totally under the conditions described here.

Also analyzed by micrograph was a reference electrode taken from a sensor that lacked an outer hydrophobic membrane. This sensor was operated continuously for 70 days prior to disassembly. The electrode showed only minimal corrosion. The absence of the hydrophobic membrane in this case allows products from the anode and cathode reactions to diffuse out of the electrolyte gel surrounding the electrodes. This results in a lower steady-state concentration of those species at the surface of the reference electrode during sensor operation. The minimal degradation of this reference electrode suggests that the unidentified reaction products may be inherently corrosive to the electrode. Alternatively, the altered distribution of ionic current between the working and counter electrodes could be responsible for the decreased corrosion in this case.

Also analyzed by micrographs were reference electrodes taken from sensors operated continuously for 10 and 70 days respectively prior to disassembly. In these two cases, reference electrodes were subjected to a constant d.c. current instead of being maintained at high impedance. The current density in each case was $5.0 \times 10^{-7}$ A/cm$^2$. One reference electrode was subjected to an anodic current, while the other electrode was subjected to a cathodic current. The polarization induced in these electrodes by the forced current was measured and found to be less than 0.5 mV. These results dramatically demonstrate the effects of DC leakage currents on reference electrode corrosion. The sensor with impressed anodic current operated for only 10 days before failing due to dendritic contact between the reference and working electrodes. The corrosion rate of the reference electrode in this sensor was much greater than that of any other configuration. The sensor with impressed cathodic current, however, demonstrated a very low corrosion rate, as indicated by the lack of surface pitting. Some surface deposits (probably silver chloride) were noticeable on this specimen.

It will be evident that there are other embodiments that are not expressly set forth above but which are clearly within the scope of the invention. Therefore the above description is to be considered exemplary only and the scope of the invention is to be defined solely by the appended claims.

**Claims**

1. A method of extending the service life of implantable sensors (2) containing corrodible electrodes (4,6,8), which comprises, in a sensor circuit including a sensor (2) having a corrodible reference electrode (4), at least one noble metal cathodic working electrode (6), and at least one noble metal anodic counter electrode (8) maintained at a low impedance, operating the sensor circuit in a first electrical configuration in which both said reference electrode (4) and said working electrode (6) are in a first electrical state, and prior to occurrence of sensor failure, switching the said sensor circuit to a second electrical configuration in which the electrical state of at least one of said reference electrode (4) and said working electrode (6) is changed to a second electrical state and continuing operation of said sensor (2) in said second electrical configuration for a period of time extending beyond the time at which said sensor (2) would have failed had said first electrical configuration of the sensor circuit been maintained.

2. A method as in Claim 1 wherein said corrodible electrode (4) is a silver or silver-impregnated electrode (4).

3. A method as in Claim 1 wherein said switching of said electrical configuration comprises increasing the input impedance at the reference electrode (4).

4. A method as in Claim 3 wherein said reference electrode (4) is also shielded to counteract the effects of stray capacitance which would cause local currents.

5. A method as in Claim 1 wherein said switching of said electrical configuration comprises, prior to formation of any dendritic structure bridging the said working (6) and reference (4) electrodes, reversing the electrical connections to said working (6) and counter (8) electrodes so that each assumes the prior operating role of the other.

6. A method as in Claim 1 wherein switching said electrical configuration comprises including in said sensor (2) a plurality of working (6) or reference (4) electrodes, with only one of said plurality of electrodes operative at any one time, with all of said electrodes adapted to be connected sequentially into the sensor circuit.

7. A method as in Claim 6 wherein there are pluralities of both said working (6) and said reference (4) electrodes, and in each plurality said electrodes are adapted to be connected sequentially into said circuit.

8. A method as in Claim 6 wherein said plurality is of said working electrodes (6) and those electrodes in said plurality which have not yet been used as the operative working electrode (6) are connected in said circuit as additional counter electrodes (8) and each operates as such until such time as it is switched into said circuit as a replacement working electrode (6).

9. A method as in Claim 8 wherein, in said plurality of said working electrodes (6), electrodes in said plurality which have not yet been used as the operative working electrode (6) are connected in said circuit as additional counter electrodes (8) and each operates as such until such time as it is switched into said circuit as a replacement working electrode (6).

10. A method as in Claim 1 wherein said switching of said electrical configuration comprises periodically changing the polarization of said working (6) and reference (4) electrodes so as to drive electrodeposited metal form working electrode (6) back to the reference electrode (4).

11. A method as in Claim 10 wherein said changing of said polarization comprises reversing the polarity of said circuit such that said reference electrode (4) becomes cathodic and said working electrode (6) becomes anodic for a period of time sufficient to pass the required number of coulombs of silver from said working electrode (6) to said reference electrode (4).

12. A method as in Claim 1 wherein said switching of said electrical configuration comprises applying to said reference electrode (4) a small, continuous cathodic current.

**Patentansprüche**

1. Verfahren zur Verlängerung der Lebensdauer implantierbarer Fühler (2), die korrodierbare Elektroden (4, 6, 8) enthalten, welches umfaßt in einem Fühlerstromkreis, der einen Fühler (2) mit einer korrodierbaren Referenzelektrode (4), zumindest einer kathodischen Arbeitselektrode (6) aus Edelmetall und zumindest einer bei einer niedrigen Impedanz gehaltenen anodischen Zählerelektrode (8) aus Edelmetall umfaßt, ein Betreiben des Fühlerstromkreises in einer ersten elektrischen Konfiguration, in welcher sowohl die Referenzelektrode (4) als auch die Arbeitselektrode (6) in einem ersten elektrischen Zustand sind, und vor dem Auftreten eines Fühlerversagens, ein Schalten des Fühlerstromkreises in eine zweite elektrische Konfiguration, in welcher der elektrische Zustand von zumindest einer der Referenzelektrode (4) und der Arbeitselektrode (6) in einen zweiten elektrischen Zustand verändert ist, und ein Fortsetzen des Betriebs des Fühlers (2) in der zweiten elektrischen Konfiguration für eine Periode an Zeit, die sich über die Zeit hinaus erstreckt, bei welcher der Fühler (2) versagt hätte, wäre die erste elektrische Konfiguration des Fühlerstromkreises beibehalten worden.

2. Verfahren nach Anspruch 1, worin die korrodierbare Elektrode (4) eine silbergefertigte oder silberimprägnierte Elektrode (4) ist.

3. Verfahren nach Anspruch 1, worin das Schalten der elektrischen Konfiguration ein Erhöhen der Eingangsimpedanz bei der Referenzelektrode (4) umfaßt.

4. Verfahren nach Anspruch 3, worin die Referenzelektrode (4) auch abgeschirmt ist, um den Auswirkungen einer Streukapazität entgegenzuwirken, welche lokale Ströme verursachen würde.

5. Verfahren nach Anspruch 1, worin das Schalten der elektrischen Konfiguration vor der Bildung irgendeiner dendritischen Struktur ein Brückenbilden bezüglich der Arbeits- (6) und Referenzelektroden (4) und ein Umkehren der elektrischen Verbindungen zu den Arbeits- (6) und Zählerelektroden (8) umfaßt, so daß jede die vorherige Betriebsfunktion der anderen übernimmt.

6. Verfahren nach Anspruch 1, worin das Schalten der elektrischen Konfiguration ein Einfügen einer Vielzahl von Arbeits- (6) oder Referenzelektroden (4) in den Fühler (2) umfaßt, wobei nur eine von der Vielzahl von Elektroden jederzeit wirksam ist und wobei alle Elektroden derart angepaßt sind, daß sie sequentiell in den Fühlerstromkreis geschaltet sind.

7. Verfahren nach Anspruch 6, worin es Vielzahlen von sowohl den Arbeits- (6) als auch den Referenzelektroden (4) gibt und wobei die Elektroden in jeder Vielzahl derart angepaßt sind, daß sie sequentiell in den Stromkreis geschaltet sind.

8. Verfahren nach Anspruch 6, worin die Vielzahl aus den Arbeitselektroden (6) besteht und die Elektroden in der Vielzahl, die noch nicht als die wirksame Arbeitselektrode (6) verwendet worden sind, als zusätzliche Zählerelektroden (8) in den Stromkreis geschaltet sind und jede als solche bis zu der Zeit arbeitet, zu der sie als eine Ersatzarbeitselektrode (6) in den Stromkreis geschaltet wird.

9. Verfahren nach Anspruch 8, worin in der Vielzahl der Arbeitselektroden (6) Elektroden in der Vielzahl, die noch nicht als die wirksame Arbeitselektrode (6) verwendet worden sind, als zusätzliche Zählerelektroden (8) in den Stromkreis geschaltet sind und jede als solche bis zu der Zeit arbeitet, zu der sie als eine Ersatzarbeitselektrode (6) in den Stromkreis geschaltet wird.

10. Verfahren nach Anspruch 1, worin das Schalten der elektrischen Konfiguration ein periodisches Verändern der Polarisation der Arbeits- (6) und Referenzelektroden (4) umfaßt, um galvanisch abgeschiedenes Metall von der Arbeitselektrode (6) zurück zu der Referenzelektrode (4) zu treiben.

**11.** Verfahren nach Anspruch 10, worin das Verändern der Polarisation ein Umkehren der Polarität des Stromkreises umfaßt, so daß die Referenzelektrode (4) kathodisch wird und die Arbeitselektrode (6) anodisch wird, und zwar für eine Periode an Zeit, die ausreicht, um die erforderliche Anzahl an Coulombs an Silber von der Arbeitselektrode (6) zu der Referenzelektrode (4) zu leiten.

**12.** Verfahren nach Anspruch 1, worin das Schalten der elektrischen Konfiguration ein Anlegen eines geringen, kontinuierlichen kathodischen Stroms an die Referenzelektrode (4) umfaßt.

**Revendications**

**1.** Procédé d'allongement de la durée de vie de capteurs implantables (2) contenant des électrodes corrodables (4, 6, 8) qui comporte, dans un circuit de capteur comprenant un capteur (2) à électrode de référence (4) corrodable, au moins une électrode de travail (6) cathodique en métal noble et au moins une contre-électrode (8) anodique en métal noble maintenue à basse impédance, en faisant fonctionner le circuit de capteur dans une première configuration électrique dans laquelle ladite électrode de référence (4) et ladite électrode de travail (6) sont toutes deux dans un premier état électrique et, avant l'apparition d'une panne de capteur, en commutant ledit circuit de capteur sur une seconde configuration électrique dans laquelle l'état électrique d'au moins une desdites électrodes de référence (4) et de travail (6) est modifié en un second état électrique et en poursuivant le fonctionnement dudit capteur (2) dans ladite seconde configuration pour une période de temps s'étendant au-delà du moment où ledit capteur (2) serait tombé en panne si ladite première configuration électrique du circuit de capteur avait été maintenue.

**2.** Procédé selon la revendication 1, dans lequel ladite électrode corrodable (4) est une électrode (4) en argent ou imprégnée d'argent.

**3.** Procédé selon la revendication 1, dans lequel ladite commutation de ladite configuration électrique comporte l'augmentation de l'impédance d'entrée au niveau de l'électrode de référence (4).

**4.** Procédé selon la revendication 3, dans lequel ladite électrode de référence (4) est également blindée pour neutraliser les effets de capacité parasite qui provoqueraient des courants locaux.

**5.** Procédé selon la revendication 1, dans lequel ladite commutation de ladite configuration électrique comporte, avant la formation de toute structure dendritique pontant lesdites électrodes de travail (6) et de référence (4), l'inversion des connexions électriques de ladite électrode de travail (6) et de ladite contre-électrode (8) de façon à ce que chacune remplisse dans le fonctionnement le rôle précédent de l'autre.

**6.** Procédé selon la revendication 1, dans lequel la commutation de ladite configuration électrique comporte l'inclusion dans ledit capteur (2) d'une pluralité d'électrodes de travail (6) ou de référence (4), une seule de ladite pluralité d'électrodes étant active en même temps, toutes les autres desdites électrodes étant destinées à être connectées l'une après l'autre au circuit de capteur.

**7.** Procédé selon la revendication 6, dans lequel il y a des pluralités tant desdites électrodes de travail (6) que desdites électrodes de référence (4) et, dans chaque pluralité, lesdites électrodes sont destinées à être connectées l'une après l'autre audit circuit

**8.** Procédé selon la revendication 6, dans lequel ladite pluralité est une pluralité desdites électrodes de travail (6) et celles des électrodes de ladite pluralité qui n'ont pas encore été utilisées en tant qu'électrode de travail active (6) sont connectées audit circuit en tant que contre-électrodes supplémentaires (8) et chacune fonctionne comme telle jusqu'à ce qu'elle soit commutée sur ledit circuit en tant qu'électrode de travail de remplacement (6).

**9.** Procédé selon la revendication 8, dans lequel, dans ladite pluralité desdites électrodes de travail (6), celles des électrodes de ladite pluralité qui n'ont pas encore été utilisées en tant qu'électrode de travail active (6) sont connectées au circuit en tant que contre-électrodes supplémentaires (8) et chacune fonctionne comme telle jusqu'à ce qu'elle soit commutée sur ledit circuit en tant qu'électrode de travail de remplacement (6).

**10.** Procédé selon la revendication 1, dans lequel ladite commutation de ladite configuration électrique comporte la modification périodique des polarisations desdites électrodes de travail (6) et de référence (4) de façon à ramener le métal électro-déposé de l'électrode de travail (6) vers l'électrode de référence (4).

**11.** Procédé selon la revendication 10, dans lequel ladite modification de ladite polarisation comporte l'inversion de la polarité dudit circuit de façon à ce que ladite électrode de référence (4) devienne cathodique et que ladite électrode de travail (6) devienne anodique pendant une durée suffisante pour faire passer le nombre de coulombs d'argent nécessaire de ladite électrode de travail (6) à ladite électrode de référence (4).

12. Procédé selon la revendication 1, dans lequel ladite commutation de ladite configuration électrique comporte l'application à ladite électrode de référence (4) d'un faible courant cathodique continu.

FIG. I

FIG. 3

EP 0 505 442 B1

FIG. 2

FIG. 5

FIG. 4

FIG. 6

12